Europäisches Patentamt

European Patent Office

Office européen des brevets

(11) Publication number: **0 093 563**
**B1**

# EUROPEAN PATENT SPECIFICATION

(45) Date of publication of patent specification: **16.06.87**

(21) Application number: **83302348.4**

(22) Date of filing: **26.04.83**

(51) Int. Cl.⁴: **A 61 K 9/08,** A 61 K 31/405, A 61 K 47/00

(54) Pharmaceutical preparation for endermic use.

(30) Priority: **30.04.82 JP 72959/82**

(43) Date of publication of application:
**09.11.83 Bulletin 83/45**

(45) Publication of the grant of the patent:
**16.06.87 Bulletin 87/25**

(84) Designated Contracting States:
**BE CH DE FR GB IT LI NL SE**

(56) References cited:
**EP-A-0 055 029**
**FR-A-1 329 357**
**GB-A-2 023 000**

**PATENTS ABSTRACTS OF JAPAN, vol. 7, no. 69 (C-158)1214r, 23rd March 1983 , JP - A - 58 4713**

**CHEMICAL ABSTRACTS, vol. 98, no. 10, 7th March 1983, page 341, no. 78067v, Columbus, Ohio, US Y. SUZUKI et al.: "Antiinflammatory and analgesic actions of IM ointment (indomethacin-1-mentholointment)"**

(73) Proprietor: **KOWA COMPANY, LTD.**
**6-29, 3-chome Nishiki**
**Naka-ku Nagoya-shi Aichi-ken (JP)**

(72) Inventor: **Inagi, Toshio**
**1-2927-2, Mihara-cho**
**Tokorozawa-shi Saitama-ken (JP)**
Inventor: **Inoue, Masayuki**
**2-17-43, Noguchi-cho**
**Higashi-Murayama-shi Tokyo (JP)**
Inventor: **Muramatsu, Toyojiro**
**1-4-9, Sayamadai**
**Sayama-shi Saitama-ken (JP)**

(74) Representative: **Webb, Frederick Ronald et al**
**G.F. Redfern & Co. Marlborough Lodge 14**
**Farncombe Road**
**Worthing West Sussex BN11 2BT (GB)**

(56) References cited:
**PATENTS ABSTRACTS OF JAPAN, vol. 7, no. 108 (C-165)1253r, 11th May 1983 , JP - A - 58 29 706**

**PATENTS ABSTRACTS OF JAPAN, vol. 5, no. 94 (C-59)766r, 19th June 1981 , JP - A - 56 36 411**

Courier Press, Leamington Spa, England.

# 0 093 563

**Description**

This invention relates to a pharmaceutical preparation containing indomethacin for endermic application.

Indomethacin is an excellent non-steroidal analgesic and antiphlogistic agent. It is, however, barely soluble in water, or in various solvents which are generally usable as bases for endermic application. Indomethacin is slightly soluble in benzyl, alcohol, tetrahydrofuran, dimethyl sulfoxide, and dimethylformamide. The indomethacin solutions produced suffer from difficulties in the formation of a preparation suitable for endermic application, from both the viewpoint of the concentration and the potency of indomethacin. Hitherto, therefore, indomethacin has been generally administered in the form of an oral preparation.

The present inventors have made many studies of preparations of indomethacin for endermic application and have already succeeded in obtaining an endermically-applicable preparation having excellent absorptivity through the skin by incorporating indomethacin in an alcohol-water system and then forming the resultant mixture into a gelled ointment, as disclosed in published Japanese Patent Application No. 10886/1981. Such a gelled ointment has been recently marketed and has been found to be very valuable in its clinical application.

The present inventors have conducted continuous research with a view to developing new dosable forms of the endermically-applicable indomethacin preparation and bases therefor. As a result, it has been discovered that certain terpenoids and phenols can enhance the solubility and stability of indomethacin in bases and hence permit indomethacin to be incorporated in a variety of bases for endermic application.

According to the present invention, therefore, there is provided a pharmaceutical preparation for endermic application, which comprises indomethacin, in an amount of 0.1—5% by weight of the preparation, at least one solubilizer selected from $C_{10}$ terpenoids and $C_{10}$ phenols in a total amount of 0.3—10% of the preparation, and an aqueous alcoholic solvent.

Suitable terpenoids having 10 carbon atoms which are useful as the solubilizer or at least one of the solubilizers in the preparations of the invention include hydrocarbon terpenes, such as limonene, pinene, camphene and cymene; alcoholic terpenes, such as citronellol, gerniol, nerol, linalol, menthol, terpineol, rosinol, borneol; and ketone-type terpenes, such as methone and camphor. Suitable phenols having 10 carbon atoms useful as the solubilizer or at least one of the solubilizers in the preparations of the invention include thymol, safrole, iso-safrole, eugenol and iso-eugenol. These solubilizers may be used singly or in combination. The content of such solubilizers when used either alone or in combination may vary depending on the content of indomethacin and the type and amount of solvent used. However, the solubilizers of the invention are capable of producing satisfactory results when incorporated in a total amount of 0.3—10% by weight of the preparation.

Suitable solvents for dissolving the indomethacin are mixed alcohol-water systems, the alcohol being, for example, ethanol or propanol.

A pharmaceutical preparation for endermic application according to the invention may be produced by dissolving indomethacin together with at least one said solubilizer in an aqueous alcoholic solvent, and, if desired, further incorporating into the solution thus formed another base for endermic application. The indomethacin is present in an amount of 0.1—5% by weight of the preparation.

Suitable forms of pharmaceutical preparation for endermic application which are obtainable in this way, include, for example, liquid preparations, ointments, gelled ointments, creams, and plasters.

Since the addition of one or more of the solubilizers of the invention in a small amount can significantly increase the solubility and stability of indomethacin in the solvents, the resultant indomethacin solutions may be incorporated in a wide variety of bases for endermic application, thus providing endermically-applicable preparations of various forms. The solubilizers used in the invention are thus extremely effective.

The invention is illustrated by the following non-limiting Examples:

## Experiment 1:

Solubility Test on Indomethacin

A large excess of indomethacin was added to each of a number of solvents, followed by addition of one of the solubilizers given in Table 1. The resultant mixture was shaken for 24 hours at 25°C and then subjected to centrifugal separation. The supernatant liquid was collected. The content of indomethacin in the supernatant liquid was determined by the UV method or the HPLC method and compared with that in a supernatant liquid having no stabilizer added thereto. The results are shown in Tables 1 and 2.

2

TABLE 1

| Solubilizer / Solvent | None (Weight dissolved) (mg/ml) | l-Menthol 3% | l-Menthol 5% | l-Menthol 10% |
|---|---|---|---|---|
| 80% Ethanol | 100 (8.0) | 140 | 200 | 270 |
| 60% Ethanol | 100 (2.4) | 160 | 350 | 440 |
| 50% Ethanol | 100 (0.75) | 350 | × | × |

Note: The figures are expressed in terms of percentage to the weight of indomethacin dissolved without any solubilizer added in their corresponding solvents.

The symbol × indicates that the solubilizer was not dissolved in the solvent.

TABLE 2

| Solubilizer / Solvent | 80% Ethanol | 60% Ethanol | 50% Ethanol |
|---|---|---|---|
| None (weight dissolved) (mg/ml) | 100 (8.0) | 100 (2.4) | 100 (0.75) |
| dl-Camphor 3% | 150 | 170 | 270 |
| dl-Camphor 5% | 190 | 230 | 400 |
| dl-Camphor 10% | 230 | 390 | × |
| Eugenol 3% | 160 | 170 | × |
| Eugenol 5% | 190 | 250 | × |
| D-limonene 3% | 170 | × | × |
| D-limonene 5% | 200 | × | × |

Note: The figures and the symbol × have the same significance as given in Table 1.

| Preparation Example 1: | (Gelled Preparation) |
|---|---|
| Indomethacin | 1.0 (wt.%) |
| l-Menthol | 3.0 |
| Propylene glycol | 12.0 |
| Carboxyvinyl polymer (CARBOPOL 934) | 1.0 |
| Diisopropanol amine | 1.0 |
| Ethanol | 40.0 |
| Purified water | Balance to 100.0 |

| Preparation Example 2: | (Liquid Preparation) |
|---|---|
| Indomethacin | 2.0 (wt.%) |
| l-Menthol | 10.0 |
| Ethanol | 45.0 |
| Aqueous ammonia (10%) | 0.2 |
| Purified water | Balance to 100.0 |

## Claims

1. A pharmaceutical preparation for endermic application, characterised in that it comprises indomethacin, in an amount of 0.1—5% by weight of the preparation, at least one solubilizer selected from $C_{10}$ terpenoids and $C_{10}$ phenols in a total amount of 0.3—10% of the preparation, and an aqueous alcoholic solvent.

2. A pharmaceutical preparation according to Claim 1, characterised in that said solubilizer, or at least one of said solubilizers, is a $C_{10}$ terpenoid selected from limonene, pinene, camphene, cymene, citronellol, geraniol, nerol, linalol, menthol, terpineol, rosinol, borneol, iso-borneol, menthone and camphor.

3. A pharmaceutical preparation according to Claim 1 or Claim 2, characterised in that said solubilizer, or at least one of said solubilizers, is a $C_{10}$ phenol selected from thymol, safrole, iso-safrole, eugenol and iso-eugenol.

4. A process for producing a pharmaceutical preparation as claimed in any one of Claims 1 to 3 comprising dissolving the indomethacin in an aqueous-alcoholic solvent, together with at least one said solubilizer selected from $C_{10}$ terepenoids and $C_{10}$ phenols.

5. A process as claimed in Claim 4, characterised in that the solution formed is incorporated into another base for endermic application.

## Patentansprüche

1. Pharmaceutisches Präparat für endermische Anwendung gekennzeichnet dadurch, daß es Indomethacin in einer Menge von 0,1 bis 5 Gew.% sowie wenigstens einen Löser ausgewählt aus $C_{10}$ Terpenoiden und $c_{10}$ Phenolen in einer Gesamtmenge von 0.3—10% des Präparates und ein wässriges alkoholisches Lösungsmittel enthält.

2. Pharmazeutisches Präparat nach Anspruch 1, dadurch gekennzeichnet, daß der Löser oder wenigstens einer der Löser ein $C_{10}$ Terpenoid ausgewählt aus Limonen, Pinen, Camphen, Cymen, Citronellol, Geraniol, Nerol, Linalol, Menthol, Terpinol, Rosinol, Borneol, Iso-borneol, Menthon und Campher ist.

3. Pharmazeutisches Präparat nach Anspruch 1 oder 2, dadurch gekennzeichnet, daß der Löser oder wenigstens einer der Löser ein $C_{10}$ Phenol ausgewählt aus Thymol, Safrol, Iso-safrol, Eugenol und Iso-eugenol ist.

4. Verfahren zur Herstellung eines pharmazeutischen Präparates nach einem der Ansprüche 1 bis 3, dadurch gekennzeichnet, daß das Indomethacin in einem wässrigen alkoholischen Lösungsmittel zusammen mit wenigstens einem Löser ausgewählt aus $C_{10}$ Terepenoiden und $C_{10}$ Phenolen gelöst wird.

5. Verfahren nach Anspruch 4, dadurch gekennzeichnet, daß die gebildete Lösung in einen anderen Grundbestandteil zur endermischen Anwendung eingemischt wird.

## Revendications

1. Préparation pharaceutique pour l'administration endermique, caractérisée par le fait qu'elle comprend de l'indométhacine, dans une proportion de 0,1 à 5% du poids de la préparation, au moins un solubilisant choisi parmi des terpénoïdes en $C_{10}$ et des phénols en $C_{10}$ dans une proportion totale de 0,3 à 10% de la préparation, et un solvant aqueux-alcoolique.

2. Préparation pharmaceutique selon la revendication 1, caractérisée par le fait que ledit solubilisant, ou au moins un desdits solubilisants, est un terepénoïde en $C_{10}$ choisi parmi le limonène, le pinène, le camphène, le cymène, le citronellol, le géraniol, le nérol, le linalol, le menthol, le terpinéol, le rosinol, le bornéol, l'iso-bornéol, le menthone et le camphre.

3. Préparation pharmaceutique selon la revendication 1 ou 2, caractérisée par le fait que ledit solubilisant, ou au moins un desdits solubilisants, est un phénol en $C_{10}$ choisi parmi le thymol, le safrole, l'iso-safrole, l'eugénol et l'iso-eugénol.

4. Procédé de production d'une préparation pharmaceutique selon l'une quelconque des revendications 1 à 3, comprenant l'étape consistant à dissoudre l'indométhacine dans un solvant aqueux-alcoolique, avec au moins un solubilisant choisi parmi des terpénoides en $C_{10}$ et des phénols en $C_{10}$.

5. Procédé selon la revendication 4, caractérisé par le fait que la solution formée est incorporée à une autre base pour l'administration endermique.